# EUROPEAN PATENT SPECIFICATION

(11) **EP 3 359 139 B1**
(45) Date of publication and mention of the grant of the patent: **07.06.2023**
(21) Application number: 15791391.4
(22) Date of filing: 09.10.2015
(51) Int. Cl.: A61K 9/24, A61K 31/192

(54) **MODIFIED RELEASE FORMULATION OF NAPROXEN SODIUM**
FORMULIERUNG MIT MODIFIZIERTER FREISETZUNG AUS NAPROXEN-NATRIUM
FORMULATION À LIBÉRATION MODIFIÉE DE NAPROXÈNE SODIQUE

(43) Date of publication of application: 15.08.2018
(73) Proprietor: Bayer HealthCare LLC, Whippany, NJ 07981 (US)
(72) Inventor: SIRIHORACHAI, Rachan, Whippany, NJ 07981 (US); ROSAR, Paul, Whippany, NJ 07981 (US)
(74) Representative: BIP Patents
(86) International application number: PCT/US2015/054873
(87) International publication number: WO 2017/062027

(56) References cited:
- WO-A1-00/04879
- WO-A1-2009/023030
- US-A- 5 756 125
- US-A1- 2014 037 725

## Description

### BACKGROUND OF THE INVENTION

### Field of the Invention

This invention relates to drug and bio-affecting compositions capable of preventing, alleviating, treating, or curing abnormal and pathological conditions of the living body by chemically altering the physiology of the body. The invention also relates to drug and bio-affecting compositions defined in terms of specific structure, as well as processes of using and preparing the same. In particular, this invention relates to anti-inflammatory compositions adapted for sustained release of naproxen and its salts in the form of bilayer tablets for oral administration.

### Description of the Prior Art

Naproxen (2-(6-methoxynaphthalen-2-yl) propanoic acid) is a well-known nonsteroidal anti-inflammatory drug (NSAID). Naproxen is a member of the 2-arylpropionic acid (profen) family of NSAIDs. Naproxen is a non-selective COX inhibitor, meaning that naproxen reversibly inhibits both the COX-1 and COX-2 enzymes, and may be used to temporarily relieve minor aches and pains (such as minor pain due to arthritis, muscular aches, backache, menstrual cramps, headache, toothache, and the common cold), as well as temporarily reduce fever.

Naproxen sodium has been the subject of numerous studies designed to evaluate the safety and efficacy of the drug for analgesic indications. Overall, results from the studies demonstrated 220 mg of naproxen sodium (equivalent to 200 mg of naproxen) to be the lowest effective dose for pain conditions in the non-prescription (over-the-counter (OTC)) setting. The evidence from dental, dysmenorrhea, headache, osteoarthritis, sore muscle, and cold studies support this dosage regimen.

Dysmenorrhea and dental studies have suggested that a better analgesic effect could be achieved in some patients with a higher (two-tablet) initial dose. In addition, it was found that patients who started with 440 mg naproxen sodium as the initial dose waited longer before re-medicating compared with those who started with an initial dose of 220 mg of naproxen sodium. Therefore it was deemed beneficial for patients to be given the option of taking an initial dose of one (220 mg) or two (440 mg) tablets of naproxen sodium.

The plasma half-life of naproxen sodium is approximately 13 hours, which supports a dosing interval of 8 to 12 hours. In comparison, the half-lives of aspirin, acetaminophen, and ibuprofen are 3.2 hours or less, which supports a notably shorter dosing interval of every 4 to 6 hours.

In light of the foregoing, naproxen sodium is made available in the United States as an OTC drug only at 220 mg. The directions for the OTC product require patients to take one dose every 8 to 12 hours while symptoms last. Although a patient may take 2 doses for the first dose, the patient must not exceed two doses in any 8 to 12-hour period or three doses in any 24-hour period. The maximum OTC-labeled dosage for naproxen sodium is 660 mg/day for 10 days.

For pain lasting at least 24 hours, the requirement of taking a dose of naproxen sodium every 8 hours, 3 times a day, may result in lower patient compliance (e.g., skipped or forgotten doses), inconsistent drug concentration in the patient's blood, and higher risk of adverse events. A dosage regimen with a lower frequency of administration should make it easy for patients to remember to take the medication and reduce "pill fatigue." Moreover, each dose can cause fluctuations in drug plasma concentration. The patient may experience more adverse events during the drug plasma concentration peak, and less pain relief during the drug plasma concentration trough. Thus, a drug plasma concentration that is more stable (fluctuates less) is desirable.

In general, drug products designed to reduce the frequency of dosing by modifying the rate of drug absorption have been available for many years. Many terms are used to describe modified-release products including extended-release, prolonged-release, controlled-release, controlled-delivery, slow-release and sustained-release. These preparations, by definition, have a reduced rate of release of active substance.

Specifically for naproxen, a technical problem for modified release formulations is caused by the drug's lowest effective dose. In particular, a modified release solid dosage form comprising a "24 hour" dose of naproxen sodium (i.e., 660 mg) is too large for some patients to comfortably swallow.

Another technical problem for modified release formulations of naproxen is caused by the relatively long half-life of naproxen. In particular, unless a formulation stops releasing the drug after about 16 to 18 hours, there will be excess naproxen sodium in the patient's blood at the time of the next dose. Thereby, extended release formulations may cause unacceptably high blood levels of naproxen.

Yet another technical problem for modified release formulations is related to the recommended loading dose of naproxen. If the modified release formulation releases less than the recommended loading dose of naproxen, the patient may not experience initial pain relief, but the patient does not have the option of taking a second dose (as the patient could have done if the first dose was a traditional 220 mg dose). Yet, if the modified release formulation releases more than the recommended loading dose of naproxen, although the patient will probably experience initial pain relief, the modified release formulation may not have enough remaining naproxen to provide the patient with effective pain relief for 24 hours.

Technical problems concerning modified release formulations of naproxen are also created because naproxen both (a) exhibits linear pharmacokinetics up to a dose of about 500 mg, and (b) fall increasingly short of linearity above 500 mg. Thus, on the one hand, a single dose of 220 mg naproxen sodium is half as effective as a single dose of 440 mg naproxen sodium. Yet, at the same time, three doses of 220 mg naproxen sodium (one dose every 8 hours) are more effective than a traditional single immediate release "24-hour" dose of 660 mg naproxen sodium.

Runkel et al., Clin. Pharmacol Ther 15:261-266, 1974, first reported that the area under the plasma drug concentration-time curve (AUC) for single doses of naproxen was reasonably linear up to 500 mg. The area under the plasma drug concentration-time curve (AUC) reflects the actual body exposure to drug after administration of a dose of the drug and is usually expressed in mg*h/L. AUC corresponds to the fraction of the dose administered that reaches the systemic circulation, and is dependent on the dose administered versus the rate of elimination of the drug from the body.

Runkel et al. was also first to report that significant deviation from AUC linearity occurred above the 500 mg dose of naproxen, and deviation progressed as the dose was further increased until the area increment between the 750 and 900 mg dose was minimal. The study authors found a disproportionate increase in the rate of elimination at elevated plasma levels, which results in lower plasma areas than might be expected from linear curves.

This initial study by Runkel et al. is often cited for these observations concerning AUC linearity of naproxen (see Runkel et al., Chem Pharm Bull 20:1457-1466, 1972; Lin et al., Clinical Pharmacokinetics 12: 402-432 (1987); and Davies et al., Clin. Phamocokinet 1997 Apr. 32 (4). 268-293).

US Patent Application Publication No. 2014/0037725 (KANNAN) describes a bilayer pharmaceutical composition comprising an immediate release component and a controlled release component of naproxen or a pharmaceutically acceptable salt thereof and the process for preparation thereof. KANNAN describes a bilayer pharmaceutical composition having a ratio of naproxen in immediate release layer to controlled release layer in the range of from about 1:2 to about 1:15. The formulation exemplified by KANNAN comprises 30% naproxen in the immediate release layer, and 70% naproxen in the extended release layer.

WO00/04879 discloses a naproxen matrix tablet, which is based on a compressed mixture of a controlled release component, and a coating on said compressed mixture, which allows for immediate release of naproxen.

WO2009/023030 discloses dosage forms providing an initial release and a second sustained release of non-steroidal-anti-inflammatory drugs, in particular ibuprofen.

US 5,756,125 discloses controlled release dosage forms composed of a naproxen layer which contains a delayed release granulate of naproxen compressed with an immediate release granulate of naproxen and an immediate release naproxen sodium layer compressed with the naproxen layer.

The technical problem outlined hereinabove is solved by the invention described hereinbelow.

### BRIEF SUMMARY OF THE INVENTION

The present invention is a formulation of naproxen having a release profile that provides immediate pain relief equivalent to taking 440 mg (or 2 tablets of 220 mg) naproxen sodium and also extended pain relief for 24 hours. In particular, the present invention is a single solid-dose combining a unique ratio of an immediate release naproxen sodium product with an extended release naproxen sodium layer. More particularly, the present invention is a single bilayer tablet that immediately releases 300-320 mg of naproxen sodium and thereafter gradually releases 450-480 mg of naproxen sodium. This product is capable of providing 24 hours of pain relief when administered as a single bilayer tablet. The single bilayer tablet provides immediate release (IR) of naproxen sodium for initial pain relief from one layer followed by continuous release of the remaining naproxen sodium from the other layer to maintain extended pain relief up to 24 hours.

### BRIEF DESCRIPTION OF THE DRAWINGS

Figure 1 is a Comparison of Dissolution Profiles of ER Layer Tablets Using Two Grades and Two Methods of Incorporation of Hypromellose at pH 7.4.
Figure 2 is a Comparison of Dissolution Profiles of ER Layer Tablets Using Two Grades and Two Methods of Incorporation of Hypromellose at pH 5.8.
Figure 3 shows the Effect of Varying the Ratio of the Naproxen in IR and ER layers on Dissolution.
Figure 4 is a Comparison of Initial Dissolution Profiles of Formulations with Different Polymer Levels Using Two Coating Systems.
Figure 5 shows Pharmacokinetic Profiles of Naproxen that compares the plasma concentration after (■) a single dose of a formulation according to the present invention comprising about 320 mg of naproxen that is immediately released, (X) an initial dose of 440 mg immediate release naproxen sodium followed 12 hours later by a second 220 mg dose of immediate release naproxen sodium, and (X) one dose of 220 mg naproxen sodium administered 3 times every 8 hours . |

### DETAILED DESCRIPTION OF THE INVENTION

The present invention is a formulation of naproxen having a release profile that provides immediate pain relief equivalent to taking 440 mg (or 2 tablets of 220 mg) naproxen sodium and also extended pain relief for 24 hours. In particular, the present invention is a single solid-dose combining a unique ratio of an immediate release naproxen sodium product with an extended release naproxen sodium layer. More particularly, the present invention is a single bilayer tablet that immediately releases 300-320 mg of naproxen sodium and thereafter gradually releases 450-480 mg of naproxen sodium. This product is capable of providing 24 hours of pain relief when administered as a single bilayer tablet. The single bilayer tablet provides immediate release (IR) of naproxen sodium for initial pain relief from one layer followed by continuous release of the remaining naproxen sodium from the other layer to maintain extended pain relief up to 24 hours. The inventors discovered that monolayer tablets incorporating a release rate controlling polymer were unsuitable to achieve the desired extended release (ER) profile.

### Granulation of naproxen Sodium

The inventors discovered that wet granulation was required to obtain a compressible form of naproxen sodium because naproxen sodium has low bulk density and poor flow characteristics. A preferred composition of the naproxen sodium granules blend is shown in Table 1.

**Table 1: Composition of Naproxen Sodium Granules**

| **Ingredient** | **%w/w** |
|---|---|
| Naproxen sodium | 60-90 |
| Cellulose, Microcrystalline | 5-15 |
| Povidone | 1.5-5 |
| | |
| Total | 100 |

Preferably, the immediate release naproxen sodium product is manufactured by wet granulating the naproxen sodium, microcrystalline cellulose, and povidone in a fluid bed granulator. These granules are used in the manufacturing of both the layers of the extended release bilayer product.

### Formulation of immediate release layer

The bi-layer extended release tablet is comprised of an IR layer and an ER layer. Preferably, the immediate release layer is made by blending the preferred naproxen sodium granules as shown in Table 1 with suitable extra-granular excipients. The extra-granular excipients are needed to help the flow properties of the granules, aid in the compression process, and avoid sticking to tooling. The weight of the immediate release tablet layer is adjusted to achieve the required ratio of naproxen sodium in the IR layer to the ER layer (the IRER ratio)..

A preferred composition of the immediate release layer used in making the bilayer extended release tablet is shown in Table 2.

**Table 2: Composition of Naproxen Sodium Immediate Release Layer**

| Ingredient | %w/w | |
|---|---|---|
| | | |
| Naproxen Sodium | 50-75 | |
| Cellulose, Microcrystalline | 10-18 | |
| Povidone | 2-5 | |
| Talc | 1-5 | |
| Magnesium Stearate | 0.2-1.5 | |

### Selection of polymer to extend release of drug

There are numerous ways of extending release of drugs (diffusion, pH dependent coatings, waxes, others). A preferred way is a hydrophilic diffusion based polymer because of its ease of processing, cost effectiveness, and batch-to-batch reproducibility. Examples of hydrophilic matrix polymers include alginic acid, carbomer, gelatin, hydroxyethylcellulose, hydroxy propylcellulose, hypromellose, methyl cellulose, polyethylene oxide, sodium carboxymethylcellulose, etc. Preferably, the polymer is a hypromellose (hydroxypropyl methylcellulose or HPMC), such as, for example, (a) hypromellose 2208 (METHOCEL^{™} K4M, Dow Chemical Company, Michigan, U.S.) and/or (b) hypromellose 2910 or (METHOCEL^{™} E4M, Dow Chemical Company, Michigan, U.S.). hypromellose polymers are widely used in the industry to extend release of drugs by creating a gel around the tablets. The drug release is controlled by the thickness of the gel, diffusion through the gel, and erosion of the gel. Two methods of incorporation of the polymer are dry and wet addition. Preferably, the dry method is used because it is simple for processing, and provides the required release profile.

The extended release layer may be tableted by direct compression of the ingredients, or from wet granulation or dry granulation of the drug and the hydrophilic polymers. Alternately the hydrophilic polymers may be added partially as part of the granulation or added externally to the granulation to form the final blend for tableting.

Preferred compositions of the extended release core tablets manufactured by both methods are shown in Table 3.

**Table 3: Composition of naproxen Sodium Extended Release Core Tablets**

| | % w/w | | | |
|---|---|---|---|---|
| Process | Dry addition | | Wet addition | |
| Ingredient | 1 | 2 | 3 | 4 |
| Granulation: | | | | |
| Naproxen sodium | 35-65 | 35-65 | 35-65 | 35-65 |
| Cellulose, microcrystalline | 3-9 | 3-9 | 3-9 | 3-9 |
| Povidone | 1-6 | 1-6 | -- | -- |
| Hypromellose 2208 or K4M | -- | -- | 4-10 | -- |
| Hypromellose 2910 or E4M | - | - | -- | 4-10 |

| Extra-granular excipients: | | | | |
|---|---|---|---|---|
| Hypromellose 2208 or K4M | 28-32 | -- | 20-25 | -- |
| Hypromellose 2910 or E4M | -- | 28-32 | -- | 20-25 |
| Lactose monohydrate | 4-12 | 4-12 | 4-12 | 4-12 |
| Silica, colloidal anhydrous | 0.2-1.5 | 0.2-1.5 | 0.2-1.5 | 0.2-1.5 |
| Magnesium Stearate | 0.1-2.0 | 0.1-2.0 | 0.1-2.0 | 0.1-2.0 |

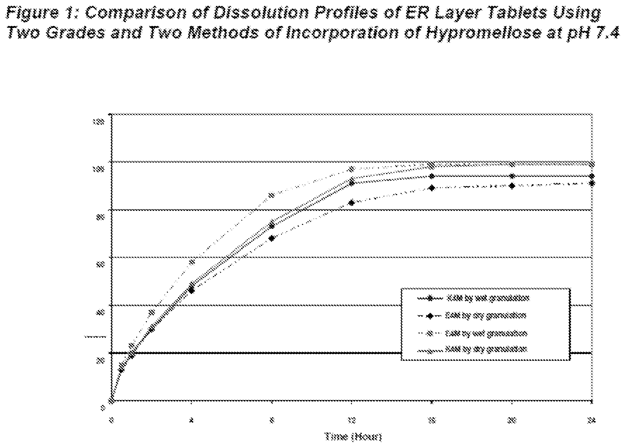

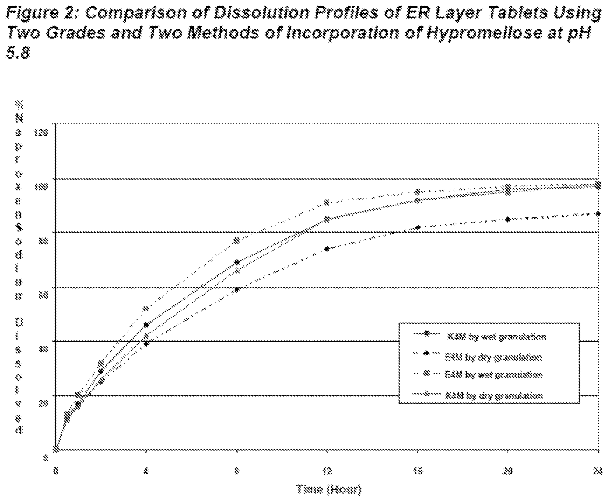

The dry addition process involves mixing of the naproxen sodium granules with the extended release polymer hypromellose, and other excipients shown in Table 3 followed by compression. The wet addition process involved adding a portion of hypromellose to replace povidone so it could be used as a binder in wet granulation. This was followed by blending the dry granules with remainder of polymer to control the release profile, extra-granular excipients, and by compression. Methods of incorporation of polymers and is shown in Figures 1 and 2, respectively.

From Figures 1 and 2, the following conclusions could be made on the dissolution data at pH 7.4 and 5.8:
Both hypromellose 2208 and 2910 yield a dissolution profile that extended release for 24 hours.
There is a difference in the dissolution profiles of hypromellose 2910 depending on the method of incorporation of polymer.
There is no significant difference in the dissolution profiles of hypromellose 2208 irrespective of method of incorporation of the polymer.

Based on the results, hypromellose 2208 is the most preferred polymer since the latter seemed to have no impact on dissolution whether added dry or wet. Dry addition of polymer was selected as the final process because (a) no new granulation was required with hypromellose as the binder; the robust naproxen granulation could be used without changes. This would be advantageous, more efficient, and cost effective since the naproxen granules could be used for both layers; and (b) equipment cleaning and processing would be much easier with dry granulation. Hypromellose when wetted swells and is slippery; clean-up could be tedious.

### Ratio of active in IR versus ER layer

The IR:ER ratio plays a critical role in the present invention. Formulations comprising different ratios of the two layers are shown in Table 4.

**Table 4: IR:ER ratio**

| IRER layer ratio | 40:60 (invention) | | 60:40 (comparison) | |
|---|---|---|---|---|
| Layer (% Naproxen sodium) | Mg/layer (mg drug) | Mg/layer (% drug) | Mg/layer (mg drug) | Mg/layer (% drug) |
| IR | 364 | 32.4 | 546 | 51.9 |
| (72.5%) | (264) | (40) | (396) | (60) |
| ER | 758 | 67.6 | 506 | 47.1 |
| (52.2%) | (396) | (60) | (264) | (40) |
| Total | 1122 | 100.0 | 1052 | 100.0 |
| | (660) | (100) | (660) | (100) |
| | | | | |

| IR:ER layer ratio | 50:50 (comparison) | | 30:70 (comparison) | |
|---|---|---|---|---|
| Layer (% Naproxen sodium) | Mg/layer (mg drug) | Mg/layer (% drug) | Mg/layer (mg drug) | Mg/layer (% drug) |
| IR | 455 | 41.8 | 273 | 23.6 |
| (72.5%) | (330) | (50) | (198) | (30) |
| ER | 632 | 58.1 | 885 | 76.4 |
| (52.2%) | (330) | (50) | (462) | (70) |
| Total | 1078 | 100.0 | 1158 | 100.0 |
| | (660) | (100) | (660) | (100) |

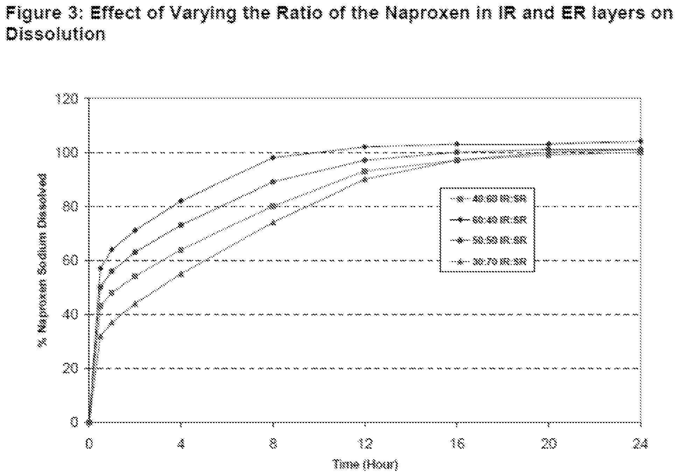

The results of the dissolution profiles with different ratios of active in IR:ER layer are shown in Figure 3. For the dissolution profiles, bilayer tablets are manufactured using the ratios presented and compressed on a rotary tablet press fitted with capsule-shaped tooling. The compression force is kept identical (about 12 kN) for all formulations and the same batch of polymer was utilized for this evaluation.

The Figure 3 shows a good correlation between the ratio of the immediate release and sustained release layers and the dissolution profile. As expected, the major difference is in the quantity of naproxen sodium released within the first 30 minutes of dissolution testing. As the naproxen sodium in the IR layer increased from 30% to 60%, the dissolution rate increased.

The 40:60 IRER layer ratio was selected as the optimal ratio for a pilot pharmacokinetic (PK) study (Bayer Impact Study #12656).

Based on all the development work for the IR and ER layers, the product was designed as a bilayer tablet with the ratio of drug in IR and ER layer being 40% and 60%, respectively. For a pilot PK study, a formulation with 30% w/w polymer was selected as the midpoint of the study. Hypromellose 2208 is selected as the rate controlling polymer. Table 5 shows the composition of the prototype for the core tablet.

**Table 5: Composition of a Tablet According to the Present Invention**

| Ingredient | %w/w | |
|---|---|---|
| Immediate-Release Layer | | |
| Naproxen Sodium Granules* | 87.9 | |
| Cellulose, Microcrystalline | 7.3 | |
| Talc | 4.1 | |
| Magnesium Stearate | 0.7 | |
| Total | 100.0 | |

| Extended-Release Layer | | |
|---|---|---|
| Naproxen Sodium Granules* | 63.2 | |
| Hypromellose 2208 | 30.0 | |
| Lactose Monohydrate | 5.5 | |
| Silica, Colloidal Anhydrous | 0.5 | |
| Magnesium Stearate | 0.7 | |
| Total | 100.0 | |

| Bilayer Caplets | | |
|---|---|---|
| Extended Release Layer | 67.6 | |
| Immediate Release Layer | 32.4 | |
| Total | 100.0 | |

| | | |
|---|---|---|
| *composition in Table 1 | | |

### Polymer Levels in the Extended Release Layer

To perform an in-vitro in-vivo correlation (IVIVC) as per the guidelines, along with the 30% w/w polymer level in the extended release layer, two other concentrations (20% & 40% w/w) were evaluated while keeping the drug ratio at 40:60 in IR and ER layers, respectively. The formulation compositions of the core tablets evaluated with various polymer levels is shown in Table 6.

**Table 6: Composition of Core Tablets with Various Polymer Levels**

| | Polymer Level (%w/w of extended release layer) | | |
|---|---|---|---|
| | 20%(comparison) | 30%(invention) | 40%(comparison) |
| Ingredient | mg/tablet | mg/tablet | mg/tablet |
| Immediate-Release Layer | | | |
| Naproxen Sodium Granules* | 320 | 320 | 320 |
| Cellulose, Microcrystalline | 26. | 26 | 26.40 |
| Talc | 15 | 15 | 15 |
| Magnesium Stearate | 2 | 2 | 2 |
| Total | 364 | 364 | 364 |

| Extended-Release Layer | | | |
|---|---|---|---|
| Naproxen Sodium Granules* | 480 | 480 | 480 |
| Hypromellose 2208 | 131 | 228 | 361 |
| Lactose, Monohydrate | 36 | 42 | 50 |
| Silica, Colloidal Anhydrous | 3 | 4 | 6 |
| Magnesium Stearate | 5 | 5 | 6 |
| Total | 654.4 | 758. | 903 |
| Total Core Weight | 1018.2 | 1122. | 1267 |

| | | | |
|---|---|---|---|
| *composition shown in Table 1 | | | |

### Coating

To improve the aesthetic appearance of the tablet and to ease swallowing, coating materials are commonly used. A comparison of the qualitative formula of two preferred coatings is shown in Table 7.

**Table 7: Comparison of the Qualitative Formula of the Opadry^{®} Coatings Coating**

| Ingredient | Opadry^{®} White YS-1-18229 | Opadry^{®} II White 49K-18329 |
|---|---|---|
| Hypromellose 2910 | Y | Y |
| Titanium Dioxide | Y | Y |
| Macrogol 8000 | Y | N |
| Carnauba Wax | N | Y |
| Polydextrose | N | Y |
| Triacetin | N | Y |

Of the two coatings, Opadry^{®} White YS-1-18229 had been used before with naproxen. It is applied as a 15.5% solids coating suspension, is compatible with the product and had no negative impact on stability.

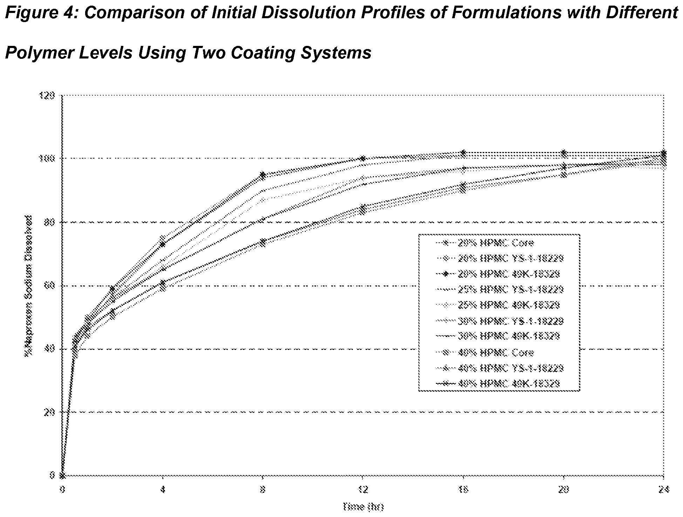

Figure 4 shows the initial dissolution profiles for formulations with different polymer levels coated with two coatings.

### Example Formulation

Based on the foregoing, exemplary formulations may be created. Table 8 shows exemplary compositions according to the present invention. A preferred formulation shown in Table 9 comprises 30% polymer in the extended release layer.

**Table 8: Exemplary Formulations**

| | HPMC level (%w/w of extended release layer) | | |
|---|---|---|---|
| | 20%(comparison) | 30%(invention) | 40%(comparison) |
| Ingredient | mg/tablet | mg/tablet | mg/tablet |
| Immediate-Release Layer | | | |
| Naproxen Sodium Granules* | 320 | 320 | 320 |
| Cellulose, Microcrystalline | 26 | 26 | 26 |
| Talc | 15 | 15 | 15 |
| Magnesium Stearate | 3 | 3 | 3 |
| Total | 364 | 364 | 364 |

| Extended-Release Layer | | | |
|---|---|---|---|
| naproxen Sodium Granules* | 480 | 480 | 480 |
| Hypromellose 2208 | 131 | 228 | 361 |
| Lactose, Monohydrate | 36 | 42 | 50 |
| Silica, colloidal anhydrous | 3 | 4 | 6 |
| Magnesium Stearate | 5 | 5 | 6 |
| Total | 654 | 758 | 903 |
| Total Core Weight | 1018 | 1122 | 1267 |

| Film-Coating | | | |
|---|---|---|---|
| Opadry White YS-1-18229 | 25 | 28 | 32 |
| Water, purified@ | -- | -- | -- |
| Polishing | | | |
| Carnauba Wax | Trace | Trace | Trace |
| Total Tablet Weight | 1044 | 1150 | 1299 |

| | | | |
|---|---|---|---|
| *Composition shown in Table 1; @evaporated during coating | | | |

**Table 9: Preferred Formulation. Each coated, bilayer tablet contains:**

| Ingredient | mg/tablet |
|---|---|
| Immediate-Release Layer | |
| Naproxen Sodium Granules* | 319.7 |
| Cellulose, Microcrystalline | 26.4 |
| Talc | 15.1 |
| Magnesium Stearate | 2.5 |
| Total | 363.8 |

| Extended-Release Layer | |
|---|---|
| Naproxen Sodium Granules* | 479.6 |
| Hypromellose 2208 | 227.8 |
| Lactose Monohydrate | 41. 8 |
| Silica, Colloidal Anhydrous | 3.8 |
| Magnesium Stearate | 5.3 |
| Total | 758.3 |

| Bilayer Caplets | |
|---|---|
| Extended Release Layer | 758.3 |
| Immediate Release Layer | 363.8 |
| Total | 1122.1 |

| Film-Coating | |
|---|---|
| Hypromellose 2910 | 33.7 |
| Titanium Dioxide | |
| Macrogol 8000 | |
| Indigo Carmine Aluminum Lake | |
| Water, purified^{@} | |
| | |
| | |
| Total | 1156 |

| | |
|---|---|
| * Composition as shown in Table 1; @ evaporated during coating; € Theoretical quantity | |

### PK Study

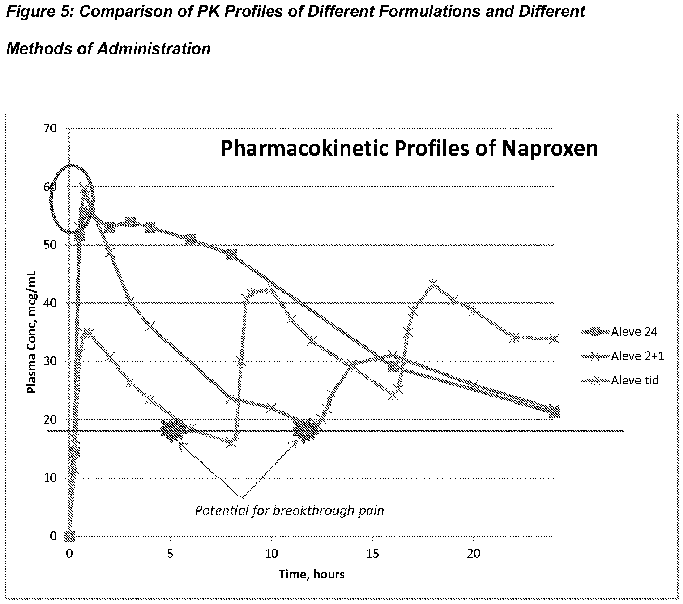

Figure 5 shows Pharmacokinetic Profiles of Naproxen that compares the plasma concentration after (■) a single dose of a formulation according to the present invention ("Aleve 24") comprising 264 mg of naproxen sodium in the immediate release layer and 396 mg naproxen sodium in the extended release layer (a 40:60 ratio), (×) an initial dose of 440 mg immediate release naproxen sodium followed 12 hours later by a second 220 mg dose of immediate release naproxen sodium ("Aleve 2+1"), and (*) one dose of 220 mg naproxen sodium every 8 hours for 3 times ("Aleve tid").

Significantly, a single dose of Aleve 24 results in: (a) an initial plasma concentration of naproxen that is equivalent to an initial dose of a 440 mg immediate release tablet, and (b) a 24-hour plasma concentration of naproxen that is continuously above the threshold for potential breakthrough pain.

At 1 hour, the total dissolution of a formulation according to the present invention ("Aleve 24") is 48% of the 660 mg dose, or 317mg of naproxen sodium. One of ordinary skill in the art would expect that 317 mg of naproxen sodium would provide a plasma level of 43 mcg/mL because of the linear pharmacokinetics of naproxen. However, the resulting plasma concentration achieved by "Aleve 24" is 55 mcg/mL, which is much higher than predicted (about 28% higher) and is closer to that observed for a regular three-times-a-day (tid) tablet dose of Aleve tablets. The Cₘₐₓ for 2 regular Aleve tablets (440mg naproxen sodium) is observed at 60 mcg/mL

Without being bound to a particular theory of the invention, it appears that the wt%/wt% ratio of naproxen sodium in the IR and ER layers plus the wt% of the polymer in the extended release layer results in the unexpected pharmacokinetic profile of formulations according to the present invention.

## Claims

1. A modified release composition for naproxen sodium comprising:
an immediate release (IR) layer comprising naproxen sodium and an extended release (ER) layer comprising naproxen sodium, the IR and ER layers both comprising naproxen sodium, naproxen sodium being present in the IR layer in an amount about 40% by weight of the total amount of naproxen sodium in the whole composition, naproxen sodium being present in the ER layer in an amount about 60% by weight of the total amount of naproxen sodium in the whole composition, the ER layer further comprising a polymer adapted to extend the release of naproxen sodium, the polymer being present in an amount of about 30% by weight of the ER layer, ,

2. The modified release composition for a drug according to claim 1, wherein the drug is present in the IR layer in an amount of 300-320 mg, and the drug is present in the ER layer in an amount of 450-480 mg.

3. The modified release composition for a drug according to claim 1, wherein the drug is present in the IR layer in an amount that is 320 mg, and the drug is present in the ER layer in an amount that is 480 mg.

4. A modified release composition for use in a method of treating pain for 24 hours, the modified release composition comprising:
an immediate release (IR) layer comprising naproxen sodium and an extended release (ER) layer comprising naproxen sodium, the IR and ER layers both comprising naproxen sodium, naproxen sodium being present in the IR layer in an amount about 40% by weight of the total amount of naproxen sodium in the whole composition, naproxen sodium being present in the ER layer in an amount about 60% by weight of the total amount of naproxen sodium in the whole composition, the ER layer further comprising a polymer adapted to extend the release of naproxen sodium, the polymer being present in an amount of about 30% by weight of the ER layer,

5. A modified release composition for use in a method of treating pain for 24 hours according to claim 4, wherein the drug is present in the IR layer in an amount that is 320 mg, and the drug is present in the ER layer in an amount that is 480 mg.

## Patentansprüche

1. Zusammensetzung mit modifizierter Freisetzung für Naproxen-Natrium, umfassend:
eine Schicht zur sofortigen Freisetzung (Immediate Release, IR), die Naproxen-Natrium umfasst, und eine Schicht mit verlängerter Freisetzung (Extended Release, ER), die Naproxen-Natrium umfasst, wobei die IR-Schicht und die ER-Schicht beide Naproxen-Natrium umfassen, wobei Naproxen-Natrium in der IR-Schicht in einer Menge von etwa 40 Gew.-% der Gesamtmenge von Naproxen-Natrium in der gesamten Zusammensetzung vorliegt, wobei Naproxen-Natrium in der ER-Schicht in einer Menge von etwa 60 Gew.-% der Gesamtmenge von Naproxen-Natrium in der gesamten Zusammensetzung vorliegt, wobei die ER-Schicht ferner ein für die Verlängerung der Freisetzung von Naproxen-Natrium geeignetes Polymer umfasst, wobei das Polymer in einer Menge von etwa 30 Gew.-% der ER-Schicht vorliegt.

2. Zusammensetzung mit modifizierter Freisetzung für einen Arzneistoff nach Anspruch 1, wobei der Arzneistoff in der IR-Schicht in einer Menge von 300-320 mg vorliegt und der Arzneistoff in der ER-Schicht in einer Menge von 450-480 mg vorliegt.

3. Zusammensetzung mit modifizierter Freisetzung für einen Arzneistoff nach Anspruch 1, wobei der Arzneistoff in der IR-Schicht in einer Menge von 320 mg vorliegt und der Arzneistoff in der ER-Schicht in einer Menge von 480 mg vorliegt.

4. Zusammensetzung mit modifizierter Freisetzung zur Verwendung bei einem Verfahren zur Behandlung von Schmerzen über einen Zeitraum von 24 Stunden, wobei die Zusammensetzung mit modifizierter Freisetzung Folgendes umfasst:
eine Schicht zur sofortigen Freisetzung (Immediate Release, IR), die Naproxen-Natrium umfasst, und eine Schicht mit verlängerter Freisetzung (Extended Release, ER), die Naproxen-Natrium umfasst, wobei die IR-Schicht und die ER-Schicht beide Naproxen-Natrium umfassen, wobei Naproxen-Natrium in der IR-Schicht in einer Menge von etwa 40 Gew.-% der Gesamtmenge von Naproxen-Natrium in der gesamten Zusammensetzung vorliegt, wobei Naproxen-Natrium in der ER-Schicht in einer Menge von etwa 60 Gew.-% der Gesamtmenge von Naproxen-Natrium in der gesamten Zusammensetzung vorliegt, wobei die ER-Schicht ferner ein für die Verlängerung der Freisetzung von Naproxen-Natrium geeignetes Polymer umfasst, wobei das Polymer in einer Menge von etwa 30 Gew.-% der ER-Schicht vorliegt.

5. Zusammensetzung mit modifizierter Freisetzung zur Verwendung bei einem Verfahren zur Behandlung von Schmerzen über einen Zeitraum von 24 Stunden nach Anspruch 4, wobei der Arzneistoff in der IR-Schicht in einer Menge von 320 mg vorliegt und der Arzneistoff in der ER-Schicht in einer Menge von 480 mg vorliegt.

## Revendications

1. Composition à libération modifiée pour le naproxène sodium comprenant :
une couche à libération immédiate (IR) comprenant du naproxène sodium et une couche à libération prolongée (ER) comprenant du naproxène sodium, les couches IR et ER comprenant toutes deux du naproxène sodium, le naproxène sodium étant présent dans la couche IR en une quantité d'environ 40 % en poids de la quantité totale de naproxène sodium dans la composition entière, le naproxène sodium étant présent dans la couche ER en une quantité d'environ 60 % en poids de la quantité totale de naproxène sodium dans la composition entière, la couche ER comprenant en outre un polymère adapté pour prolonger la libération de naproxène sodium, le polymère étant présent en une quantité d'environ 30 % en poids de la couche ER.

2. Composition à libération modifiée pour un médicament selon la revendication 1, dans laquelle le médicament est présent dans la couche IR en une quantité de 300 à 320 mg, et le médicament est présent dans la couche ER en une quantité de 450 à 480 mg.

3. Composition à libération modifiée pour un médicament selon la revendication 1, dans laquelle le médicament est présent dans la couche IR en une quantité qui est de 320 mg, et le médicament est présent dans la couche ER en une quantité qui est de 480 mg.

4. Composition à libération modifiée pour utilisation dans un procédé de traitement de la douleur pendant 24 heures, la composition à libération modifiée comprenant :
une couche à libération immédiate (IR) comprenant du naproxène sodium et une couche à libération prolongée (ER) comprenant du naproxène sodium, les couches IR et ER comprenant toutes deux du naproxène sodium, le naproxène sodium étant présent dans la couche IR en une quantité d'environ 40 % en poids de la quantité totale de naproxène sodium dans la composition entière, le naproxène sodium étant présent dans la couche ER en une quantité d'environ 60 % en poids de la quantité totale de naproxène sodium dans la composition entière, la couche ER comprenant en outre un polymère adapté pour prolonger la libération de naproxène sodium, le polymère étant présent en une quantité d'environ 30 % en poids de la couche ER.

5. Composition à libération modifiée pour utilisation dans un procédé de traitement de la douleur pendant 24 heures selon la revendication 4, dans laquelle le médicament est présent dans la couche IR en une quantité qui est de 320 mg, et le médicament est présent dans la couche ER en une quantité qui est de 480 mg.
